# EUROPEAN PATENT SPECIFICATION

(11) **EP 3 610 885 B1**
(45) Date of publication and mention of the grant of the patent: **06.07.2022**
(21) Application number: 19461505.0
(22) Date of filing: 22.01.2019
(51) Int. Cl.: A61K 38/18, A61K 9/00, A61K 47/59, A61P 27/02, A61P 27/06

(54) **COMPOSITION FOR THE INTRAVITREAL ADMINISTRATION OF THERAPEUTIC PROTEIN**
ZUSAMMENSETZUNG ZUR INTRAVITREALEN VERABREICHUNG VON THERAPEUTISCHEM PROTEIN
COMPOSITION POUR L'ADMINISTRATION INTRAVITRÉENNE DE PROTÉINES THÉRAPEUTIQUES

(30) Priority: 16.08.2018 PL 42666218
(43) Date of publication of application: 19.02.2020
(73) Proprietor: Pomorski Uniwersytet Medyczny w Szczecinie, 70-204 Szczecin (PL)
(72) Inventor: Machalinski, Boguslaw, 71-806 Szczecin (PL); Dabkowska, Maria, 71-050 Szczecin (PL); Roginska, Dorota, 71-118 Szczecin (PL)
(74) Representative: Witek, Rafal

(56) References cited:
- WO-A1-2015/168347
- WO-A2-2009/046446
- US-A1- 2009 202 505
- US-A1- 2019 142 964
- RAYMOND IEZZI ET AL: "Dendrimer-based targeted intravitreal therapy for sustained attenuation of neuroinflammation in retinal degeneration", BIOMATERIALS, ELSEVIER SCIENCE PUBLISHERS BV., BARKING, GB, vol. 33, no. 3, 6 October 2011 (2011-10-06), pages 979-988, XP028112171, ISSN: 0142-9612, DOI: 10.1016/J.BIOMATERIALS.2011.10.010 [retrieved on 2011-10-14]
- LEYUAN XU ET AL: "Dendrimer Advances for the Central Nervous System Delivery of Therapeutics", ACS CHEMICAL NEUROSCIENCE, vol. 5, no. 1, 26 November 2013 (2013-11-26), pages 2-13, XP055439825, US ISSN: 1948-7193, DOI: 10.1021/cn400182z
- ANNA MACHALINSKA ET AL: "Long-Term Neuroprotective Effects of NT-4-Engineered Mesenchymal Stem Cells Injected Intravitreally in a Mouse Model of Acute Retinal Injury", INVESTIGATIVE OPTHALMOLOGY & VISUAL SCIENCE, vol. 54, no. 13, 23 December 2013 (2013-12-23), page 8292, XP055619145, US ISSN: 1552-5783, DOI: 10.1167/iovs.13-12221
- MIGNANI SERGE ET AL: "Expand classical drug administration ways by emerging routes using dendrimer drug delivery systems: A concise overview", ADVANCED DRUG DELIVERY REVIEWS, ELSEVIER, AMSTERDAM, NL, vol. 65, no. 10, 13 February 2013 (2013-02-13), pages 1316-1330, XP028737356, ISSN: 0169-409X, DOI: 10.1016/J.ADDR.2013.01.001
- MARIA DABKOWSKA ET AL: "Electrostatic complex of neurotrophin 4 with dendrimer nanoparticles: controlled release of protein in vitro and in vivo", INTERNATIONAL JOURNAL OF NANOMEDICINE, vol. Volume 14, 1 August 2019 (2019-08-01) , pages 6117-6131, XP055619143, DOI: 10.2147/IJN.S210140

## Description

The invention relates to a composition for the intravitreal administration of a therapeutic protein for the treatment and prevention of neurodegenerative diseases of the retina.

Retinal degenerative diseases are an important clinical problem. Retinal degeneration occurs in about 25 million people worldwide. It is estimated that in 2020 the percentage of people blind due to the retinal degenerative diseases in the European population will come to 19.1%.

These diseases are progressive: within a few years of diagnosis they lead to progressive blindness and permanent disability. Advanced age is the primary risk factor for development of many degenerative retinal disorders leading to visual impairment and blindness, such as glaucoma, retinal pigmented degeneration and age-related macular degeneration (AMD) [Xu H, Chen M, Forrester JV: Para-inflammation in the aging retina. Prog Retin Eye Res 2009, 28(5):348-68, Hims MM Ophthalmology 2003, 37:109-125].

The diseases are conditioned by many factors, including environmental and genetic, and are currently incurable due to the lack of causative therapy [Ophthalmology. 2009; 116:57-65]. Functional changes of the retina, i.e. lowering the visual acuity, contrast sensitivity, color perception, delayed adaptation to night conditions [Jackson GR, Owsley C: Scotopic sensitivity during adulthood. Vision Res 2000, 40(18):2467-73. Owsley C: Aging and Vision. Vision Res 2011, 51(13):1610-22.] result from the accumulation of disorders at the molecular level, including the nerve cell layer, the pigment epithelium and the Bruch's retinal membrane.

Despite significant progress in the field of treatment of neurodegenerative diseases, no effective therapies have been developed to inhibit neurodegenerative processes and to induce repair mechanisms in the damaged retina of the eye. It is believed that a number of factors influence the retinal degeneration, such as: decrease in the production of neurotrophic factors, increase in oxidative stress or changes in retinal cells due to metabolic stress leading to inflammation [LaVail, M.M. Proc.Natl.Acad.Sci.USA 89,11249-11253, Paskowitz, D.M. IOVS 2007,48,430-437, Machalińska Retina IOVS 2013,54,13,8292]. Despite numerous reports on the protective role of neurotrophins on the retina of the eye [Progress in retinal and Eye Research 29(2010):443-465, Drug Discovery Today, 13, 3/4, p.144; Macromolecular Biosci. 2012, 12, 608-620; Drug development and industrial pharmacy, 2013, 39(11):1599-1617], until now the long-term effect of Neurotrophin 4 (NT-4) on biology of the retina has not been thoroughly investigated.

Despite many prospective applications of low molecular weight proteins such as neurotrophin-4, the development of effective technologies enabling targeted therapy involving the transport of the above-mentioned protein on a biocompatible carrier, and then its release at a specific site, has not been sufficiently determined. This is mainly due to insufficient knowledge of the physicochemical fundamentals controlling the transport and adsorption of the carrier-protein system and the lack of interdisciplinary approach to research teams involved in the studies of kinetic aspects of protein adsorption/desorption processes on/from potentially biocompatible carriers under conditions when they are subjected to intense shear forces as they occur *in vivo.* In addition, there are no scientific reports on the formation, under defined conditions of diffusion transport, of biocompatible stable films with adsorbed proteins, with different surface charges and conformation of the adsorbed macromolecules, on surfaces of varying degrees of roughness.

Dendrimers are large, three-dimensional, symmetrical organic polymers obtained by cyclically repeated series of polymerization reactions (often referred to as cascade polymerization) around a core molecule leading to the addition of successive layers to a spherical-shaped polymer molecule. PAMAM-type (i.e. polyamidoamine) dendrimers, produced by adding successive layers of - CH₂CH₂CONHCH₂CH₂N units obtained from the sequential attachment of methyl acrylate and ethylene diamine to a single core molecule (most commonly NH₃ or ethylenediamine), have been discovered and described by Tomalia D.A. *et al.* (Tomalia DA et al. "Starburst Cascade Polymers: Molecular-Level Control from Size, Shape, Surface Chemistry, Topology, and Flexibility from Atoms to Macroscopic Matter", Angew. Chem. Int. Ed. Engl. 29:138 (1990)). Numerous methods for obtaining them are also known in the art (see PCT/US83/02052 and U.S. Patent Nos. 4,507,466, 4,558,120, 4,568,737, 4,587,329, 4,631,337, 4,694,064, 4,713,975, 4,737,550, 4,871,779 and 4,857,599). Controlled and stepwise layered growth of this structure leads to obtaining of subsequent generations of polymers with precisely defined sizes, molecular masses and the number of functional groups (in particular amine residues) on their surface that determine cationic nature of the polymer molecule.

Attachment of six layers of -CH₂CH₂CONHCH₂CH₂N units to the core ethylenediamine molecule results in obtaining of the sixth-generation polymer PAMAM G6.0 (CAS number 163442-69-1) with a molecular weight of 58046.11 AMU and 256 surface amine groups. This compound is commercially available until the 1990s.

WO 2015/168347 discloses PAMAM dendrimer nanoparticles linked to at least one biologically active agent.

Raymond lezzi et al. Biomaterials, vol. 33, no. 3, pages 979-988 XP028112171 describes intravitreal administration of PAMAM dendrimers conjugated to fluocinolone acetate for treatment of retinal degeneration.

Leyuan Xu et al. ACS Chemical Neuroscience, vol. 5, no. 1, pages 2-13, XP055439825 describes drug delivery via dendrimers.

US 2009/202505 describes intravitreal administration of NT-4 (recombinant expression vectors) for the treatment of macular degeneration.

Anna Machalińska et al, Investigative Ophthalmology & Visual Science vol. 54, no. 13, page 8292, XP055619145 discloses neuroprotective effects of NT-4 injected intravitreally and treatment of acute retinal injury. WO 2009/046446 describes drug-nanoparticle formulation including inter alia PAMAM.

Mignani Serge et al. Advanced Drug Delivery Reviews, vol. 65, no. 10, pages 1316-1330, XP028737356 provides review on dendrimer drug delivery where different PAMAM dendrimer generations are mentioned.

The object of the invention is to provide substances and forms of medicaments suitable for intravitreal administration of ophthalmic drugs, in particular for the treatment or prevention of neurodegenerative diseases of the retina.

The subject of the invention is a composition for intravitreal administration of a therapeutic protein characterized in that it comprises a therapeutic protein and a polyamidoamine dendrimeric polycation combined electrostatically with the therapeutic protein, whereinthe therapeutic protein is neurotrophin-4 (NT-4) and the polyamidoamine dendrimeric polycation is a PAMAM dendrimer, preferably PAMAM G 6.0.

Another subject of the invention is an electrostatic complex consisting of PAMAM poliamidoamine dendrimeric polycation, preferably PAMAM G 6.0, and neurotrophin-4 (NT-4).

Another subject of the invention is the electrostatic complex defined above for use in medicine, preferably for use in the prophylaxis or treatment of eye diseases. Preferably, the electrostatic complex for use according to the invention is characterized in that the eye disease is a retinal degenerative disease, in particular selected from the group comprising of age-related macular degeneration, gyrate atrophy, retinitis pigmentosa and glaucoma. Preferably, the electrostatic complex for use according to the invention is characterized in that it is used in a medicament intended for intravitreal administration of neurotrophin-4, in particular in a form of medicament with a sustained release of neurotrophin-4.

### Detailed description of the invention

According to the invention, a universal nanometer-scale biocarrier was obtained, intended for medical applications, in particular from inducing retinal regeneration (improving its function), preferably in neurodegenerative diseases. Commercially-available branched polyelectrolytes, sixth generation poliamidoamine dendrimers (6G) were used for preparation of the biocarrier for transport and controlled release of neurotrophic factors, i.e. neurotrophin 4, after intravitreal administration. The trophic factor-carrier binding force was determined, allowing for development of a system for local, long-term and continuous protein release for 60 days. In an *in vivo* mouse model, it has been shown that the nanostructures proposed according to the invention are suitable for advantageous tissue delivery of pharmacologically active biological compounds that have an effect on the function of the damaged retina at the cellular and molecular level, including its pro-regenerative and anti- degenerative properties.

The generated electrostatic complex of neurotrophin 4 - PAMAM 6G dendrimer was tested in disorders of the retina function in the model of selective, acute pigment epithelium damage and its role in the mechanism of "post-injury" neuroregeneration of the retina. A detailed assessment of the kinetics of functional and morphological changes within the damaged retina in mice was performed by administering the NT-4 protein-PAMAM dendrimer complex to the environment of the retina. Next, the mouse retinal regeneration process was evaluated after induction of damage selectively involving the retinal pigment epithelium (RPE) of the eye.

In the first step, a selective damage of the mouse retinal pigment epithelium was induced with sodium iodate (NaIO3) according to an established protocol. Next, the electrostatic complex of protein-dendrimer was administered intravitreally and a detailed analysis of the kinetics of changes in the animal retina was performed. The examined parameters included intravital functional (ERG, electroretinography) and morphological (SD-OCT, spectral domain optical coherence tomography) assessment at 3 selected time points - prior to and on days 7th, 14th and 28th post- administration of the damaging factor (6 mice of each sex per time point). At the same time points the kinetics of changes in the retinal expression of the NT-4 neurotrophic factor at the protein level were examined in the obtained retinal preparations, which correlated with functional changes of the retina and endogenous regenerative response from the retinal nerve. The applied research methods allowed for a more precise determination of the therapeutic effect of the protein-carrier system used.

The experimental work performed in *in vitro* and *in vivo* model allowed for:
- obtaining a stable electrostatic complex NT-4 protein - PAMAM 6G dendrimer that allows for the continuous quantitatively-controlled desorption of neurotrophins within the vitreous and their diffusion to cells located in the retinal pigment epithelium for 28 days,
- determining the concentration ranges of the adsorbed proteins and the effect of surface concentration of the biocompatible layer of neurotrophins on the retina of the murine eye,
- determining the protein-dendrimer binding force by performing protein desorption measurements in both phosphate buffer solution and the gel system that is the vitreous of the murine eye; the performed measurements allowed for determining the equilibrium constants for the protein-dendrimer binding and determined the diffusion rate of neurotrophins from the vitreous to the retina,
- characterizing the stability of the generated biocompatible neurotrophin layers.

### Example 1. Preparation of electrostatic complexes consisting of PAMAM G 6.0 and neurotrophin-4 (NT-4).

### NT-4 solutions

### Preparation of neurotrophin-4 solutions

Commercially purchased NT-4 protein (recombinant human neurotrophin 4, CF, purchased from R&D Systems, cat. number 268-N4-250ug/CF) was used to obtain dendrimer-neurotrophin 4 bionanocarriers. 500 mg/L protein solutions were obtained by dissolving NT-4 in phosphate buffer, then the solution was filtered using filters (centrifree ultrafiltration device, Merck Millipore, cat. no. 4104). In order to minimize errors in determining the exact concentration of neurotrophin-4 solutions prepared in PBS, their values were determined before dilution by two complementary spectrophotometric techniques: BCA (abcam, AB207002) and absorbance measurement at 280 nm. Prior to each experiment, the original neurotrophin-4 solution was diluted to 5-100 mg/L. All NT-4 protein solutions were prepared in a buffered saline solution without calcium and magnesium ions (PBS fluid) with osmolarity of 270-300 mOsmol/L and pH 7.4±0.2 (Biomed Lublin company).

The composition of the PBS fluid: sodium chloride NaCl 8 g/L, potassium chloride KCl 0.2 g/L, disodium hydrogen phosphate Na₂HPO₄ x 12 H₂O 2.9 g/L; potassium diphosphate KH₂PO₄ 0.2 g/L.

### Studies of neurotrophin-4 solutions

Determination of physicochemical parameters, i.e. diffusion coefficient, particle diameter, microelectrophoretic mobility and zeta potential of proteins in solutions is important to assess the adsorptive reversibility or its absence from the surface of PAMAM polymers and is crucial in the development of the effective Neurotrophin 4 - PAMAM drug. The diffusion coefficient, as well as NT-4 size were determined in the solution by dynamic light scattering (DLS) using Zetasizer Nano ZS device (Malvern). The physicochemical characterization of NT-4 molecules in PBS solutions with ionic strength of 0.15 M was performed for two pH values of 3.5 and 7.4. Over 33 days the changes of NT-4 diffusion coefficients (temp. 293 K) were evaluated, as well as the hydrodynamic diameter in the PBS solution.

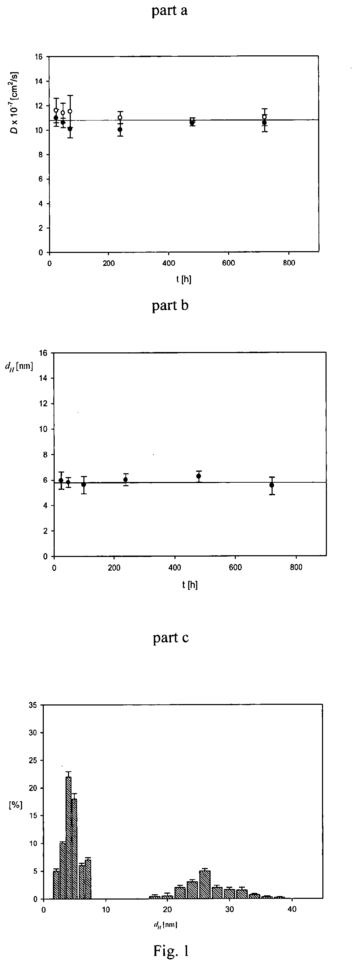

Fig. 1 shows the stability of NT-4 protein as a dependence of the diffusion coefficient (part a)/hydrodynamic diameter (part b) over time. Points represent experimental values from DLS measurements for 200 µg/mL NT-4, 0.15 M PBS: (•) pH 7.4 and (∘) pH 3.5. Part C shows a histogram with the distribution of the hydrodynamic diameter of NT-4 molecules in 0.15 M PBS solution at pH=7.4. Error bars represent standard deviation calculated from 6 measurements.

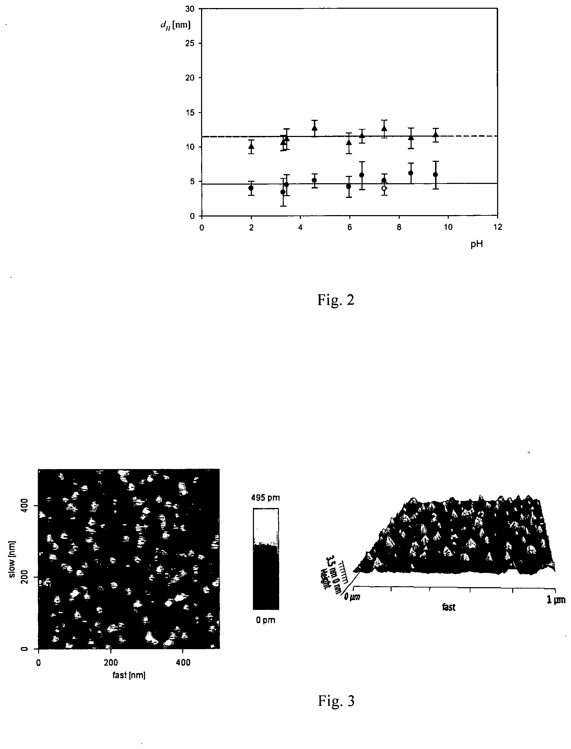

Fig. 2 shows the hydrodynamic diameter of NT-4 protein determined by DLS method as a function of pH change. Points represent experimental results obtained by dynamic light scattering for NT-4 solutions at concentration of 200 µg/mL, 0.15 M PBS: (•, o) after ultrafiltration, and (▲) without ultrafiltration.

In addition, the size of NT-4 molecules adsorbed on the surface of mica was evaluated with NanoWizard AFM atomic force microscope (JPK Instruments AG). The mica was immersed in the NT-4 solution in PBS, 1 mg/L, the adsorption time was 1 minute, then it was thoroughly rinsed with ultrapure water for 1 minute to remove non-adsorbed protein molecules from its surface. The sample thus prepared was air-dried prior to the analysis. Next, the coverage of mica surface with molecules of the adsorbed neurotrophin was determined using the AFM microscope and amounted to 1 mg/m². The diameter of the adsorbed NT-4 molecule was determined using the Loco's Shire v.2.8.24.0 and ImageJ programs.

Fig. 3 shows AFM microscope photographs showing NT-4 protein molecules adsorbed on the surface of mica.

As can be seen, the NT-4 molecules have a spherical shape that resembles globular proteins. Their diameter from the AFM measurement is 5.1±2 nm (after filtration) as shown in Fig. 3. AFM measurements of the size of neurotrophin adsorbed on the solid surface are in good agreement with the measurements of this protein in the PBS solution.

The electrophoretic mobility of NT-4 molecules in PBS solution was measured by the microelectrophoresis technique to determine the electrokinetic charge that the protein has in the PBS electrolyte solution. Then it was converted into the zeta potential of the neurotrophin particle which allowed us to determine at which pH values we can adsorb the NT-4 protein on the surface of the PAMAM dendrimer using electrostatic interactions.

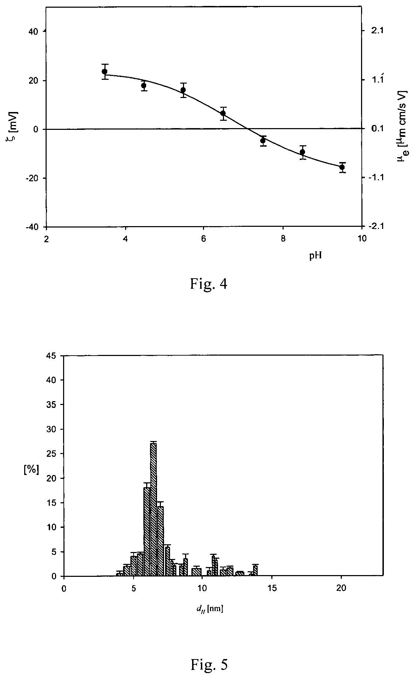

Fig. 4 shows the NT-4 zeta potential/electrophoretic mobility (ξ/µₑ) as a function of pH of the solution in 0.15 M PBS solution at the temperature of 293 K.

As can be seen, electrophoretic mobility of neurotrophin-4 in pH 3.5 is 0.95 µm cm/s V which corresponds to a zeta potential of 19 mV, while at pH 9.5 it is -1.12 µm cm/s V which corresponds to a zeta potential of -20 mV. In the PBS solution at pH 7.4, the zeta potential has a slightly negative value of -5 mV.

### PAMAM dendrimer solutions

Commercially-available sixth generation PAMAM 6G dendrimers (Sigma Aldrich, cat. number 536717) were used to obtain the dendrimer-neurotrophin 4 complexes. PAMAM solutions at a concentration of 50 mg/L were obtained by diluting the initial concentrated dendrimer solution 1000-fold in PBS phosphate buffer, followed by filtering of the solution using filters (0.1 µm lowest binding membrane for protein solutions, PVDF Durapore, Merck Millipore, product number SLVV033RS). The size (hydrodynamic diameter) of the PAMAM 6 polymer was determined in the solution by dynamic light scattering (DLS) using Zetasizer Nano ZS device (Malvern). Fig. 5 shows a histogram of the distribution of PAMAM 6.0 dendrimer hydrodynamic diameter as determined by the DLS method. The points indicate experimental results obtained by dynamic light scattering for PAMAM solutions at a concentration of 200 µg/mL in 0.15 M PBS at pH 7.4.

As can be seen, PAMAM molecule diameter from the DLS measurement is 7±0.2 nm (after filtration) which is shown in Figure 5. Histogram 5 shows a very monodispersed dendrimer size distribution with 85% of all PAMAM molecules having a diameter fluctuating within 7±1 nm.

The electrophoretic mobility, as well as the zeta potential of PAMAM molecules were determined in 0.15 M PBS solution using microelectrophoresis technique (LDV) to determine their uncompensated electrokinetic charge. The electrophoretic mobility of PAMAM dendrimer at pH 7.4 was 1.16±0.19 µm cm/s V, which corresponds to the zeta potential of 22.2±3.7 mV.

### Preparation and characterization of the dendrimer-neurotrophin-4 electrostatic complex

The protein was adsorbed on the surface of PAMAM G 6.0 dendrimer using electrostatic interactions. Adsorption of neurotrophin-4 on the dendrimer carrier was performed in a PBS solution (composition as above) with ionic strength of 0.15 M and pH 7.4 in a 2-ml eppendorf tube. Under the applied conditions, neurotrophin-4, as it results from its zeta potential of -5 mV, having a negative electrokinetic charge, diffuses onto the surface of the PAMAM dendrimer of a zeta potential of 22 mV, thus of a positive electrokinetic charge.

Adsorption of the protein on the nanocarrier proceeded according to the following procedure:
i) the microelectrophoretic mobility of NT-4 protein and PAMAM dendrimer was measured (by the method described above),
ii) NT-4 layers on the dendrimer surface were formed by mixing equal volumes of the solutions, i.e. 0.5 ml of the NT-4 solution at a concentration of 40 mg/L with 0.5 ml of the PAMAM solution at a concentration of 100 mg/L,
iii) adsorption proceeded for 15 min which was enough time to reach the maximum coverage/full NT-4 monolayer on the dendrimer nanoparticle [Adamczyk, Z.; Bratek-Skicki, A.; Dqbrowska, P.; Nattich-Rak, M. Mechanisms of fibrinogen adsorption on latex particles determined by zeta potential and AFM measurements. Langmuir 2012, 28, 474-485].
iv) The entire procedure was performed in eppendorf tubes with intensive stirring at 293 K. Hydrodynamic diameter of the generated electrostatic complex of 20 µg/mL NT-4 - 50 µg/mL PAMAM was measured in a PBS solution at pH 7.4 using the DLS method. It was 13.5±5 nm.

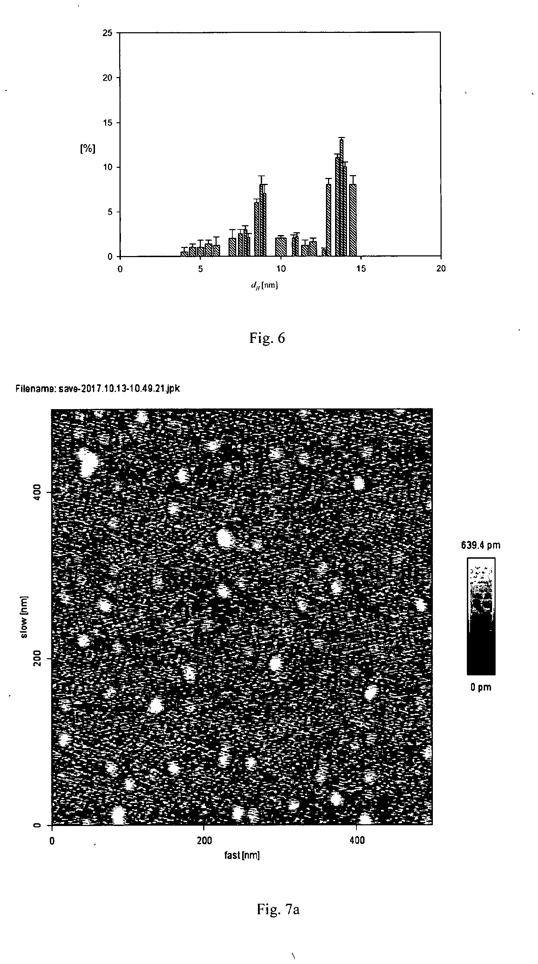

Fig. 6 shows a histogram of the distribution of NT-4-PAMAM 6.0 electrostatic complex hydrodynamic diameter as determined by the DLS method. The points indicate experimental results obtained by dynamic light scattering for NT-4 (20 µg/mL) - PAMAM (50 µg/mL) solutions in 0.15 M PBS at pH 7.4.

The zeta potential of the electrostatic complex 20 µg/mL NT-4 - 50 µg/mL PAMAM measured by the microelectrophoresis technique in 0.15 M PBS pH 7.4 was 12±8 mV.

Additionally, the diameter of the NT-4-PAMAM electrostatic complex adsorbed on the mica surface was tested using the NanoWizard AFM atomic force microscope (JPK Instruments AG). Mica was immersed in NT-4-PAMAM solution in 0.15 M PBS at pH 7.4, the adsorption time was 15 minutes, then rinsed thoroughly with ultrapure water for 1 minute to remove non-adsorbed molecules of the protein or the electrostatic complex from its surface. The sample thus prepared was air-dried prior to the analysis. Next, using the AFM microscope, the coverage of mica surface with molecules of the adsorbed electrostatic complex was determined using the AFM microscope and amounted to 0.5 mg/m². The diameter of the adsorbed NT-4-PAMAM electrostatic complexes was determined using the Loco's Shire v.2.8.24.0 and ImageJ programs.

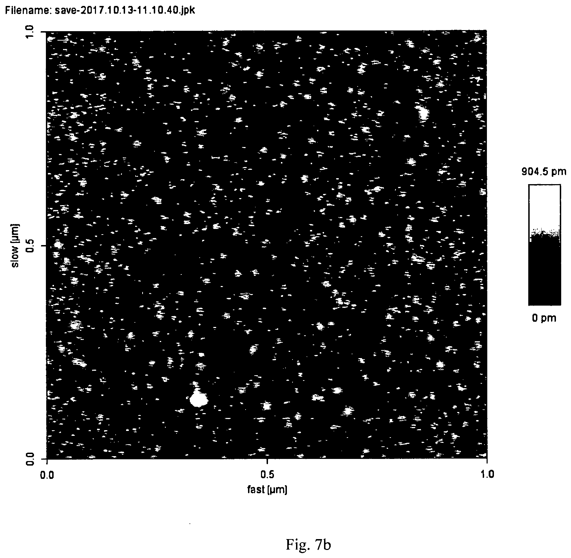

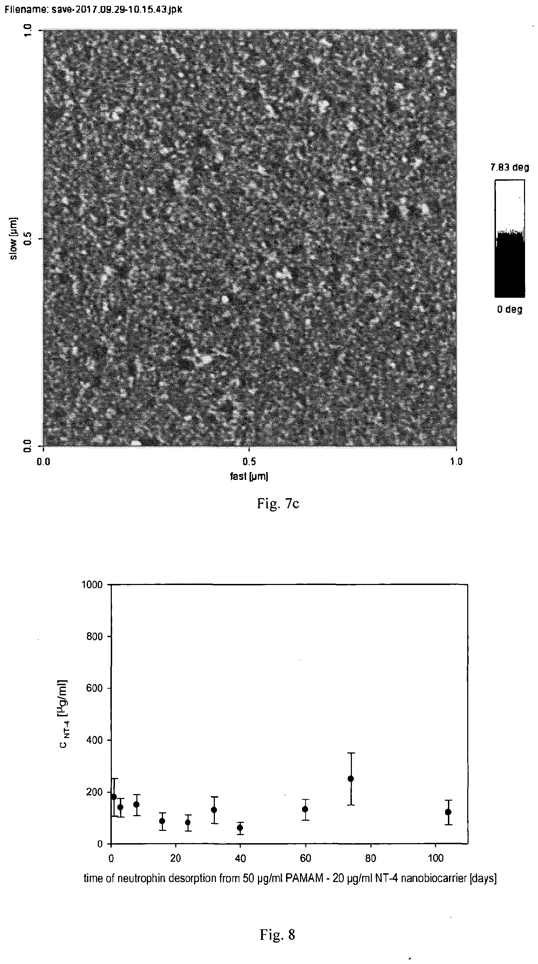

Fig. 7 shows AFM microscope photographs showing NT-4-PAMAM complexes adsorbed on the surface of mica.

As can be seen, NT-4-PAMAM complexes have a spherical shape and their diameter obtained in the AFM measurements is 18±5 nm (after filtration) as shown in Fig. 7. AFM measurements of the size of NT-4-PAMAM complexes adsorbed on the surface of mica are in good agreement with the DLS measurements in the volume of the solution.

### Kinetics of neurotrophin-4 desorption from the dendrimer-neurotrophin-4 complexes in PBS solution

The study of neurotrophin-4 desorption from the PAMAM nanoparticles over 3 months was performed with the technique of loss of solution concentration in a time unit, using ELISA method (R&D Systems test codes DY992, DY990, DY994, DY999, DY995, WA126, DY006, DY268). For this purpose, following NT-4 adsorption on the PAMAM dendrimer, the obtained solutions were ultrafiltered on filters with a cut-off of 30 kDa in a PBS solution. The molecular weight of a single neurotrophin-4 molecule is 14 kDa, therefore after filtration all NT-4 proteins non-bound to the dendrimer are in the filtrate. The NT-4 concentration in the resulting filtrate was determined by ELISA. This technique was used to monitor the NT-4 release from the dendrimer as a function of time (Fig. 8) in a 0.15 M PBS solution at pH 7.4.

Fig. 6 shows the kinetics of protein desorption from the NT-4-PAMAM nanobiocarrier as a function of time.

The kinetics profile of NT-4 release proves that the use of the 50 µg/ml PAMAM - 20 µg/ml NT-4 nanobiocarrier guarantees continuous release of the protein at concentration levels of 200±50 pg/ml for at least 102 days.

### Example 2. The use of electrostatic complexes consisting of PAMAM G 6.0 and neurotrophin-4 (NT-4) in the treatment of eye diseases.

### Animals

C57BL6/J/*cmdb* mice (Center for Experimental Medicine, Medical University of Bialystok) of both sexes, weighing 27-30 g were used in the study. Mice procedures were initiated after obtaining a positive opinion of the local ethics committee for animal experiments in Poznan (resolution no. 53/2017 of 06/10/2017). The animals were kept in an individually ventilated cage system (IVC rack: BIO A.S. Vent II Ehret, Emmendingen, Germany), with a twelve-hour day/night cycle and *ad libitum* access to food and water.

### The model of acute damage to the retina of the eye

NaIO₃ selectively destroys the retinal pigment epithelium and then the photoreceptor layer adhering thereto. The nature of the damage is similar to the degenerative changes in the retina of the eye, observed in the case of diseases occurring in humans, e.g. age-related macular degeneration, gyrate atrophy or retinitis pigmentosa. Therefore, this model is useful in research on the development of therapeutic strategies in the treatment of degenerative ocular diseases in humans.

Prior to the procedure of retinal damage caused by toxic effects of NaIO₃, the mice were anesthetized by intraperitoneal administration of ketamine (40 mg/kg) and xylazine (4 mg/kg) solution (both preparations from Biowet, Pulawy, Poland). In addition, the cornea was anesthetized with a 0.5% proxymetacaine hydrochloride solution (Alcaine, Alcon, Fort Worth, TX, USA). Then, animals were administered with NaIO₃ into the retro-orbital venous sinus in a single dose of 25 mg/kg.

### Intravitreal administration of PAMAM and the PAMAM-NT4 complex

Prior to the procedure, mice were anesthetized by intraperitoneal administration of ketamine (40 mg/kg) and xylazine (4 mg/kg) solution. In addition, the cornea was anesthetized with a 0.5% proxymetacaine hydrochloride solution (Alcaine, Alcon, Fort Worth, TX, USA). The PAMAM-NT-4 complexes and the PAMAM dendrimer were administered by intravitreal injection into the opposite eyes. The volume of the administered fluid was µl. The control group consisted of animals that received the NaIO₃ injection alone.

### Spectral domain optical coherence tomography (SD-OCT)

Spectral domain optical coherence tomography (SD-OCT) allows for intravital and non-invasive assessment of the retinal morphology. The study was performed prior to and 7 and 28 days after the retinal damage induced by NaIO₃ toxicity and the intravitreal administration of the dendrimer-neurotrophin 4 complex (n=12 mice) using the Bioptigen Envisu^{™} R2200-HR SD-OCT system (Leica Microsystems, Wetzlar, Germany).

Prior to the procedure, mice were anesthetized by intraperitoneal administration of ketamine (40 mg/kg) and xylazine (4 mg/kg) solution. Then, in order to dilate the pupils, a 1% atropine solution (Atropinum Sulfuricum WZF, Polfa Warszawa, Poland) was applied to the conjunctival sac. Throughout the study, mice were treated with gel eye drops (Systane Gel Drops; Alcon, Fort Worth, TX, USA) to maintain corneal transparency and provide good image quality.

Morphometric analysis of retinal thickness was performed using InVivoVue software and the *calliper* tool (Bioptigen, Leica Microsystems, Wetzlar, Germany), measuring the distance between the first hyperreflexive layer, i.e. the vitreoretinal border, and the pigment epithelium. Measurements were taken in all quadrants of the retina at a distance of 450 µm from the center of the optic disc.

It was assumed that the mean value of all measurements taken was the total thickness of the retina of the eye. To visualize the changes in the retinal thickness of mice in time kinetics, the data are presented as a percentage, individually estimated for each animal on the basis of measurements taken on days 7 and 28 relative to the value obtained prior to the damage induced by NaIO₃ toxicity and the intravitreal administration of the dendrimer-neurotrophin 4 complex, which was taken as 100%.

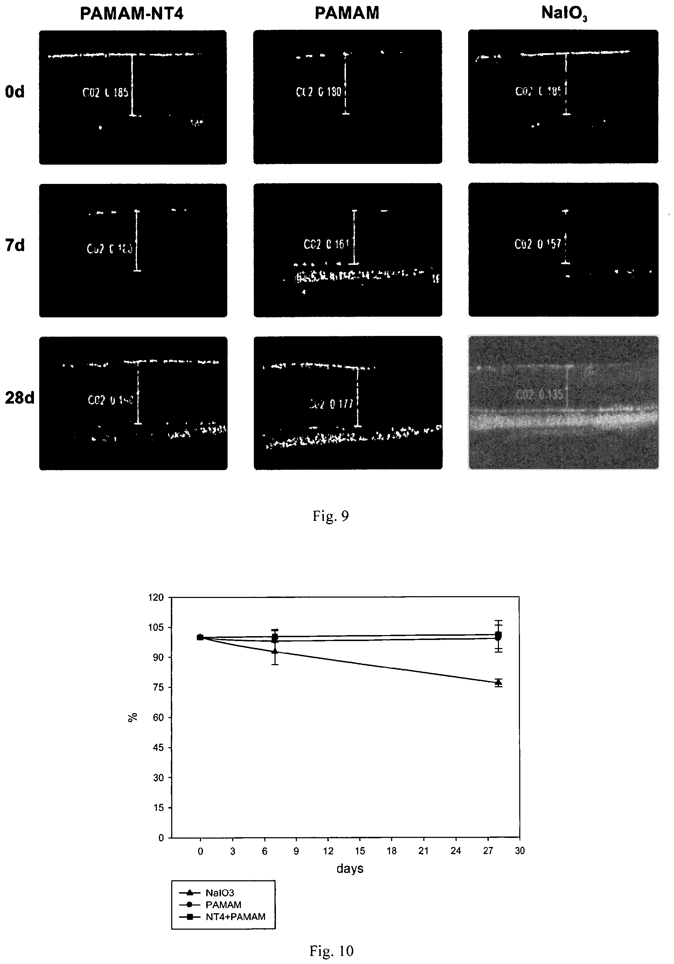

Morphometric analysis of the retinal scans revealed that a significant decrease in the retinal thickness occurs in the course of retinal damage induced by NaIO₃ toxicity. Complete retinal regeneration was observed in the groups of mice that received intravitreal injection of the PAMAM-NT4 complex and the PAMAM dendrimer (Figs 9 and 10).

Fig. 9 shows representative tomograms of murine retinas prior to and on days 7^{th} and 28^{th} post-administration of PAMAM-NT4, PAMAM and NaIO₃ obtained by the SD-OCT method.

Fig. 10 shows the kinetics of changes in murine retinal thickness prior to and on days 7^{th} and 28^{th} post-administration of PAMAM-NT4, PAMAM and NaIO₃, determined on the basis of morphometric measurements of the retinal scans obtained by the SD-OCT method. The values obtained on day 0 were taken as 100%.

### Electroretinography (ERG)

Electroretinography (ERG) allows for assessment of the bioelectrical function of the retina in an objective and non-invasive way. The study was performed prior to and 7 and 28 days after the retinal damage induced by NaIO₃ toxicity and the intravitreal administration of the dendrimer-neurotrophin 4 complex (n=12 mice), in scotopic and photopic conditions, using full field ERG (ffERG, Ganzfeld).

Following overnight dark adaptation, a 1% atropine solution was applied to the conjunctival sac in order to dilate the pupil. Next, mice were anesthetized by intraperitoneal administration of ketamine (40 mg/kg) and xylazine (4 mg/kg) solution. To minimize discomfort of the animals caused by the use of contact electrodes, the cornea was anesthetized with 0.5% proxymetacaine hydrochloride solution (Alcaine; Alcon, Fort Worth, TX, USA). The following types of leads were used to record the ERG: (i) active electrode - corneal, in the form of contact lens with built-in gold ring, (ii) needle reactive electrode - reference, placed subcutaneously in the forehead area of the mouse head, between the ears, (iii) needle reactive electrode - grounding, inserted subcutaneously at the tail base of the animal (all electrodes types were from LKC Technologies, Gaithersburg, MD, USA).

The ERG recording was obtained using the UTAS BigShot^{™} system (LKC Technologies, Gaithersburg, MD, USA). The protocol consisted of the following steps:
1. Rod response, obtained in nocturnal adaptation, by stimulation with a flash of white light of low intensity (24 dB neutral filter). 8 recordings were averaged, recorded in 8-second intervals.
2. Rod-cone response, initiated in nocturnal adaptation, by stimulation with a flash of white light with maximum intensity (standard flash, 0 dB filter). 2 recordings were averaged, recorded with a 28-second interval.
3. Oscillatory potential, induced in nocturnal adaptation, by stimulation with a single flash of white light with maximum intensity (standard flash, 0 dB filter).
4. Cone response, obtained after a 10-minute day adaptation (30 cd/m² light intensity) and a standard flash stimulation (0 dB filter). 8 recordings were averaged, recorded in 1-second intervals.

In all steps, a band-stop filter (notch filter) was used to reduce line interference.

The bioelectrical function analysis of the retina was based on the b-wave amplitude, which was measured from the trough of the a-wave to the peak of the b-wave or, in the absence of the a-wave, from the isoelectric line to the peak of the b-wave.

To visualize the electrophysiological changes of the retina in time kinetics, the data are presented as a percentage, individually estimated for each animal on the basis of the b-wave amplitude measurement taken on days 7 and 28 relative to the value obtained prior to the damage induced by NaIO₃ toxicity and the intravitreal administration of the dendrimer-neurotrophin 4 complex, which was taken as 100%.

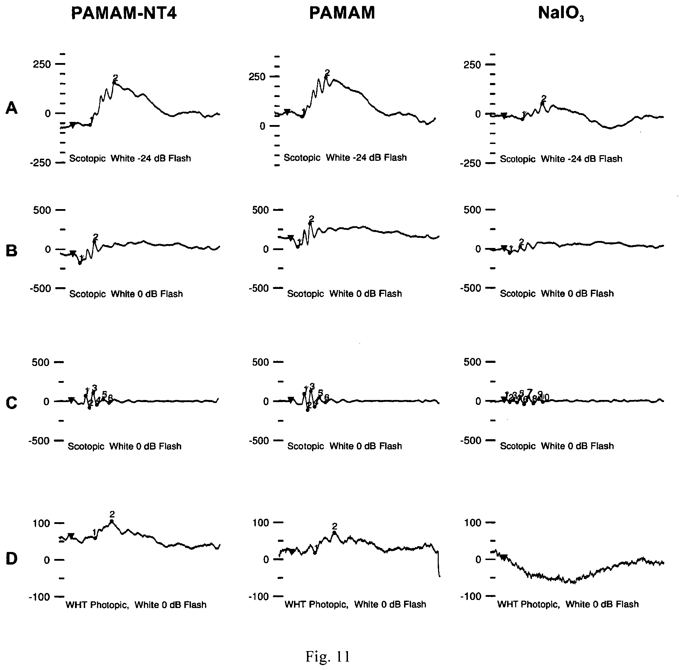

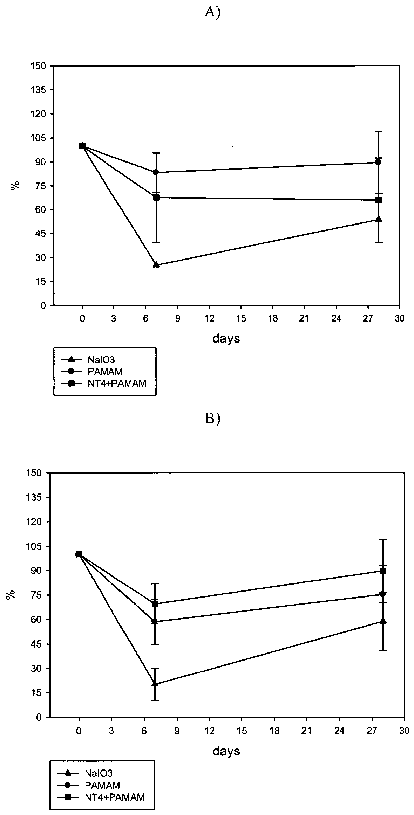

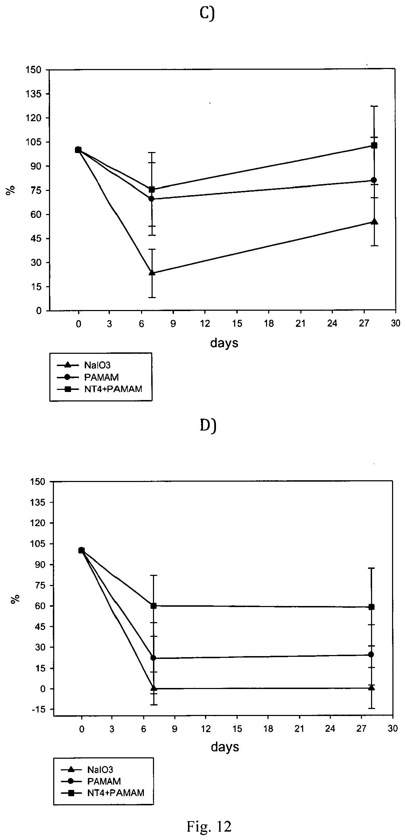

Analysis of ERG recordings in time kinetics showed that in all experimental groups the b-wave amplitude of the rod, rod-cone and cone responses, as well as the sum of oscillatory potentials decreases in relation to the measurements obtained prior to the NaIO₃-induced retinal damage and the intravitreal injection of nanostructures. However, in the group of mice administered with the PAMAM-NT-4 complex, the retinal bioelectrical activity was much better preserved (Figs 11 and 12).

Fig. 11 shows representative electroretinograms of the rod (A), rod-cone (B) and cone (D) responses, as well as the sum of oscillatory potentials (C) obtained on the day 28^{th} post-administration of PAMAM-NT4, PAMAM and NaIO₃.

Fig. 12 shows the kinetics of retinal bioelectrical activity changes of the rod (A), rod-cone (B) and cone (D) responses, as well as the sum of oscillatory potentials (C) prior to and on days 7^{th} and 28^{th} post-administration of PAMAM-NT4, PAMAM and NaIO₃. The measurements obtained on day 0 were taken as 100%.

### Protein isolation

Prior to and 7, 14 and 28 days after the to the retinal damage induced by NaIO₃ toxicity and the intravitreal administration of the dendrimer-neurotrophin 4 complex, some mice were euthanized by cervical dislocating and eyeballs were harvested, from which the vitreous and the neurosensory part of the retina along with the pigment epithelium were separately dissected. Total protein was isolated from the obtained biopsies using a commercially available PARIS kit (Thermo Fisher Scientific, Waltham, USA). In the first isolation step, the frozen tissues were transferred to 300 µl of Cell Dissruption Buffer, enriched with a mix of protease (Protease Inhibitor Coctail, set III, Merck Millipore, Billerica, MA, USA) and phosphatase (1 mM sodium fluoride and 2 mM sodium orthovanad (Sigma-Aldrich, St. Louis, MO, USA)) inhibitors. Next, the tissues were ground using Omni TH handheld homogenizer equipped with hard tissue tips (Omni International, Kennesaw, GA, USA). To complete the lysis process, the homogenates were left on ice for an additional 10 min, followed by centrifugation with the following parameters: 4°C, 14,000 rpm, 12 min. The obtained supernatant was subjected to spectrophotometric protein concentration measurement by the Bradford method (Sigma-Aldrich, St. Louis, MO, USA). The protein extract was used to assay the neurotrophin-4 concentration by ELISA.

### Kinetics of neurotrophin-4 desorption from the dendrimer-neurotrophin-4 complex in the vitreous and retinas of the murine eyes

In order to test the effect of the complex on improving murine retinal function, levels of NT-4 concentrations in the murine eye prior to and after intravitreal administration of 50 µg/ml PAMAM - 20 µg/ml NT-4 in a volume of 1 µl were determined. The characterization was started by testing the neurotrophin-4 expression at the level of the receptor organ such as the eye, after intravitreal NaIO₃ administration to induce retinal damage, therefore both retinas and vitreous were isolated from mice according to the procedure described in section 2.1.6.

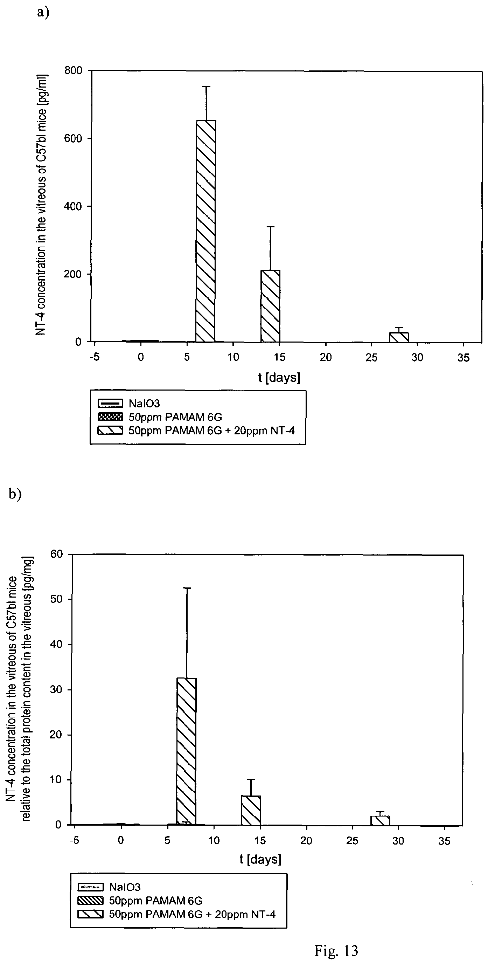

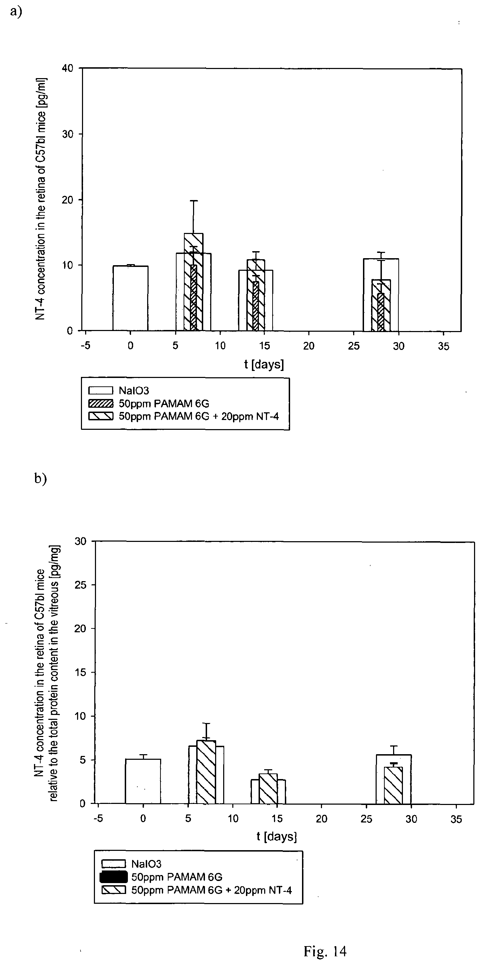

NT-4 concentrations in murine vitreous on day 0, i.e. without NaIO₃ administration, 7 days post-administration and 28 days post-administration of the retinal damaging substance were determined using the ELISA technique (abcam, R&D Systems, BD Biosciences) as shown in Figs 13 and 14.

Fig. 13 shows the evaluation of NT-4 protein concentration levels in murine vitreous after the administration of NaIO₃, PAMAM dendrimer, NT-4-PAMAM complex on days 0, 7^{th}, 14^{th} and 28^{th} post-administration. a) The NT-4 protein concentration is given in the unit [pg/ml]. b) The NT-4 protein concentration is given in the unit [pg/mg] i.e. it is normalized based on the mass of the total protein content in individual murine vitreous.

On the basis of the obtained results, NT-4 concentrations in murine vitreous were determined without NaIO₃ administration (3.0±1.9 pg/ml, in the figure as day 0), 7 days post-administration (2.3±0.3 pg/ml) and 28 days post-administration (0 pg/ml). Figure 8 b) presents experimental data on the above-mentioned NT-4 concentrations converted into neurotrophin amounts relative to the total protein content in the vitreous. The total protein content in the vitreous was determined using the Bradford method.

NT-4 concentrations in the vitreous of damaged murine eye were measured by the same method after intravitreal administration of PAMAM carrier alone on days 7^{th}, 14^{th} and 28^{th} post-administration. The results obtained in the vitreous using the ELISA technique are shown in Figure 13 a) and reached 3.1±1.7 pg/ml on day 7^{th} post-administration and 0 pg/ml on day 28^{th} post-administration. Figure 13 b) presents experimental data on the above-mentioned NT-4 concentrations converted into neurotrophin amounts relative to the total protein content in the vitreous.

In addition, NT-4 concentrations in the vitreous of damaged murine eye were measured after the intravitreal administration of the 50 µg/ml PAMAM - 20 µg/ml NT-4 complex on days 7^{th}, 14^{th} and 28^{th} post-administration. The results obtained in the vitreous using the ELISA technique are shown in Figure 13 a) and reached 654±100 pg/ml on day 7^{th} post-administration and 28±15 pg/ml on day 28^{th} post-administration of. Figure 13 b) presents experimental data on the above-mentioned NT-4 concentrations converted into neurotrophin amounts relative to the total protein content in the vitreous.

Fig. 14 shows an evaluation of the NT-4 protein levels in murine retinas after administration of NaIO₃, PAMAM dendrimer, NT-4-PAMAM complex on days 7^{th}, 14^{th} and 28^{th} post-administration. a) The NT-4 protein concentration is given in unit [pg/ml]. b) The NT-4 protein concentration is given in unit [pg/mg], i.e. it is normalized based on the mass of the total protein content in individual murine retinas.

On the basis of the obtained results, NT-4 concentrations [pg/ml] in murine retinas were determined without NaIO₃ administration (9.9±0.2 pg/ml, in the Figure 14 as day 0), 7 days post-administration (11.8±1.2 pg/ml) and 28 days post-administration (11.1±1.1 pg/ml). Figure 14 b) presents experimental data on the NT-4 concentrations in murine retinas converted into neurotrophin amounts relative to the total protein content in the vitreous. The total protein content in the vitreous was determined using the Bradford method.

NT-4 concentrations in the retinas of damaged murine eye were measured by the same method after intravitreal administration of PAMAM carrier alone on days 7^{th}, 14^{th} and 28^{th} post-administration. The results obtained in the vitreous using the ELISA technique are shown in Figure 14 a) and reached 10.0±2 pg/ml on day 7^{th} post-administration and 5.8±1.5 pg/ml on day 28^{th} post-administration. Figure 14 b) presents experimental data on the above-mentioned NT-4 concentrations converted into neurotrophin amounts relative to the total NT-4 protein content in the vitreous.

NT-4 concentrations in the retinas of damaged murine eye were also measured after the intravitreal administration of the 50 µg/ml PAMAM - 20 µg/ml NT-4 complex on days 7^{th}, 14^{th} and 28^{th} post-administration. The results obtained by ELISA in the vitreous are shown in Figure 14 a) and reached values of 14.9±5 pg/ml on day 7 after administration and of 7.9±3 pg/ml on day 28 after administration. Figure 14 b) presents experimental data on the above-mentioned NT-4 concentrations converted into neurotrophin amounts relative to the total NT-4 protein content in the vitreous.

## Claims

1. A composition for intravitreal administration of a therapeutic protein, **characterized in that** it comprises a therapeutic protein and a polyamidoamine dendrimeric polycation combined electrostatically with the therapeutic protein, wherein the therapeutic protein is neurotrophin-4 (NT-4) and the polyamidoamine dendrimeric polycation is a PAMAM dendrimer, preferably PAMAM G 6.0.

2. An electrostatic complex consisting of PAMAM poliamidoamine dendrimeric polycation, preferably PAMAM G 6.0, and neurotrophin-4 (NT-4).

3. The electrostatic complex as defined in claim 2 for use in medicine, preferably for use in the prophylaxis or treatment of eye diseases.

4. The electrostatic complex for use of claim 3, **characterized in that** the eye disease is a retinal degenerative disease, in particular selected from the group consisting of age-related macular degeneration, gyrate atrophy, retinitis pigmentosa and glaucoma.

5. The electrostatic complex for use of claim 3, **characterized in that** it is used in a medicament intended for intravitreal administration of neurotrophin-4, in particular in the form of medicament with a sustained release of neurotrophin-4.

## Patentansprüche

1. Zusammensetzung zur intravitrealen Verabreichung eines therapeutischen Proteins, **dadurch gekennzeichnet, dass** sie ein therapeutisches Protein und ein mit dem therapeutischen Protein elektrostatisch kombiniertes dendrimeres Polyamidoamin-Polykation umfasst, wobei es sich bei dem therapeutischen Protein um Neurotrophin-4 (NT-4) und bei dem dendrimeren Polyamidoamin-Polykation um PAMAM-Dendrimer, vorzugsweise PAMAM G 6.0, handelt.

2. Elektrostatischer Komplex, der aus dem dendrimeren Polyamidoamin-Polykation PAMAM, vorzugsweise PAMAM G 6.0, und Neurotrophin-4 (NT-4) besteht.

3. Elektrostatischer Komplex gemäß Anspruch 2 zur Verwendung in der Medizin, vorzugsweise zur Verwendung in der Prophylaxe oder Behandlung von Augenkrankheiten.

4. Elektrostatischer Komplex zur Verwendung gemäß Anspruch 3, **dadurch gekennzeichnet, dass** die Augenkrankheit eine Netzhautdegenerationserkrankung ist, die insbesondere aus der Gruppe ausgewählt ist, die aus altersbedingter Makuladegeneration, Atrophia gyrata, Retinitis pigmentosa und Glaukom besteht.

5. Elektrostatischer Komplex zur Verwendung gemäß Anspruch 3, **dadurch gekennzeichnet, dass** er in einem Medikament verwendet wird, das für die intravitreale Verabreichung von Neurotrophin-4 bestimmt ist, insbesondere in Form eines Medikaments mit verzögerter Freisetzung von Neurotrophin-4.

## Revendications

1. Composition pour une administration intravitréenne d'une protéine thérapeutique, **caractérisée en ce qu'**elle comprend une protéine thérapeutique et un polycation dendrimérique polyamidoamine combiné de manière électrostatique avec la protéine thérapeutique, dans laquelle la protéine thérapeutique est la neurotrophine-4 (NT-4) et le polycation dendrimérique polyamidoamine est un dendrimère PAMAM, de préférence PAMAM G 6.0.

2. Complexe électrostatique constitué par un polycation dendrimérique polyamidoamine PAMAM, de préférence PAMAM G 6.0, et la neurotrophine-4 (NT-4).

3. Complexe électrostatique tel que défini dans la revendication 2 pour son utilisation en médecine, de préférence pour son utilisation dans la prophylaxie ou le traitement de maladies de l'œil.

4. Complexe électrostatique pour son utilisation selon la revendication 3, **caractérisé en ce que** la maladie de l'œil est une maladie dégénérative de la rétine, en particulier sélectionnée dans le groupe constitué par la dégénérescence maculaire liée à l'âge, l'atrophie gyrée, la rétinite pigmentaire et le glaucome.

5. Complexe électrostatique pour son utilisation selon la revendication 3, **caractérisé en ce qu'**il est utilisé dans un médicament destiné à l'administration intravitréenne de neurotrophine-4, en particulier sous la forme d'un médicament avec une libération prolongée de neurotrophine-4.
